(19) Europäisches Patentamt
European Patent Office
Office européen des brevets

(11) **EP 3 912 547 A1**

(12) **EUROPEAN PATENT APPLICATION**

(43) Date of publication:
**24.11.2021 Bulletin 2021/47**

(51) Int Cl.:
*A61B 5/00* (2006.01)     *A61B 5/04* (2006.01)
*A61B 5/0476* (2006.01)     *G06F 3/01* (2006.01)
*A61F 2/72* (2006.01)     *A61B 5/0488* (2006.01)

(21) Application number: **20175553.5**

(22) Date of filing: **19.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **TOYOTA JIDOSHA KABUSHIKI KAISHA TOYOTA-SHI, AICHI-KEN 471-8571 (JP)**
• **CAMLIN ITALY S.r.l. 43123 Parma (IT)**

(72) Inventors:
• **AMBECK-MADSEN, Jonas**
  **1140 Brussels (BE)**
• **BELLOTTI, Andrea**
  **1140 Brussels (BE)**
• **DI LIBERTO, Giovanni M.**
  **43123 Parma (IT)**
• **ASCARI, Luca**
  **43123 Parma (IT)**
• **COLUCCIELLO, Alessia**
  **43123 Parma (IT)**

(74) Representative: **Cabinet Beau de Loménie**
**158, rue de l'Université**
**75340 Paris Cedex 07 (FR)**

(54) **COMPUTER-IMPLEMENTED METHOD AND DATA-PROCESSING DEVICE FOR OBTAINING CONTINUOUS SIGNALS FROM BIOPOTENTIAL SIGNALS**

(57) The present invention concerns a computer-implemented method for obtaining continuous signals from biopotential signals. This method comprises the separation of confounding non-brain components from the biopotential signals by a statistical correlation analysis algorithm, such as a Canonical Correlation Analysis algorithm, to obtain denoised neural signals, and the decoding of the continuous signals from the denoised neural signals. The present invention also relates to a data-processing device (1) configured to execute this method.

**FIG.2**

**Description**

TECHNICAL FIELD

The disclosure relates to a computer-implemented method for obtaining continuous signals from biopotential signals, as well as to a data processing device comprising a processor configured to perform the computer-implemented method, a non-invasive brain-computer interface system comprising such a data processing device and a biopotential electrode array connected to the data processing device, and to a computer program or readable medium comprising instructions that, when executed by a computer, cause the computer to implement the abovementioned computer-implemented method.

BACKGROUND

Most current Human-Machine-Interfaces (HMI) rely on motor actions for inputting human commands. Even vocalization of instructions for voice input requires in fact such motor actions at the level of throat, jaw and mouth muscles. However, the nerve transmission delay between the brain activity triggering those motor actions and the actual motor actions introduces a not-insignificant time lag, which can be critical in some applications, such as, for instance, driving a vehicle. In other applications, such as controlling prosthetic devices, the HMI actually aims to replace the connection between nerves and muscles, and would thus also benefit from direct brain input.

In order to obtain a HMI with direct input from brain activity anticipating and/or replacing the actual motor action, that is, a so-called Brain-Machine-Interface (BMI), it has already been propose to decode motor actions from electroencephalographic (EEG) signals. For instance, classification-based decoding of motor actions from EEG signals has been proposed in European patent application publication EP 3 556 429 A1, in US patent US 9,824,607 B1, and in international patent application publication WO 2015/003118 A1. However, such classification-based decoding provides discrete outputs, whereas a continuous output may be advantageous for tasks such as e.g. vehicle steering.

Another solution, proposed for instance in US patent application publication US 2014/0058528 A1 is to fit decoders for 2D/3D kinematics from data recorded when users imagined performing a motor task. However, solutions based on imagined tasks have significant shortcomings. For example, they may not be able to anticipate motor actions. Moreover, confounding non-brain components in the EEG signals represent an obstacle for fitting generalized decoding models applicable without pre-training even, for example, to impaired users.

SUMMARY

A first object of the disclosure is that of providing a computer-implemented method for reliably obtaining continuous signals from biopotential signals.

According to a first aspect of the disclosure, this may be achieved with the steps of separating confounding components from the biopotential signals by using a statistical correlation analysis algorithm, such as a Canonical Correlation Analysis algorithm, to obtain denoised neural signals, and decoding the continuous signals from the denoised neural signals.

By identifying and removing the confounding components from the biopotential signals, it is possible to obtain denoised neural signals that can be more reliably correlated to continuous signals, even with an anticipating time shift allowing short-term prediction of the continuous signals.

The continuous signals may in particular be continuous motor action command signals, that is, command signals related to an intended motor action of the user. Alternatively, however, they may be any other sort of continuous signals possibly embedded within the biopotential signals, such as e.g. brain-originated signals related to a level of attention and/or comfort.

The statistical correlation algorithm may be performed using a model based on an existing dataset of biopotential signals and correlated motion, vision, acoustic and/or other biopotential data. The correlated motion data may include EMG data, motion sensor data and/or visual motion capture data. The biopotential signals and/or correlated other biopotential data may comprise EEG, skin conductivity response (SCR) and/or electrocardiographic (ECG) data, besides the EMG data

The step of decoding continuous signals from the denoised neural signals may be performed using a Multiple Linear Regression model, which may in particular correlate continuous signals to denoised neural signals within an anticipating time window, so as to obtain short-term predictions of continuous signals from current denoised neural signals.

The computer-implemented method may comprise a step of filtering the biopotential signals before the step of separating the confounding components thereof.

The computer-implemented method may further comprise a step of operating a machine, such as, e.g. a vehicle (including personal mobility devices such as wheelchairs), a robotic manipulator, a prosthetic device and/or a rehabilitation device, using the continuous signals as command signals. A human user may thus output continuous commands to operate the machine through the biopotential signals.

Alternatively or additionally to the step of operating the machine using the continuous signals, the method may comprise a step of verifying whether the continuous signals correspond to continuous commands within a set of acceptable continuous commands. It may thus be verified, eventually in advance, whether a continuous command to be output by a human user through e.g. a motor action is acceptable, so as to be able to otherwise take adequate preventative and/or correcting measures.

Alternatively or additionally to the step of operating the machine using the continuous signals, the method may comprise a step of comparing the continuous signals to a response of a machine operated by a human user generating the biopotential signals. When testing the machine it is thus possible to evaluate whether it responds appropriately to the user's intentions.

A second aspect of the present disclosure relates to a data processing device comprising a processor configured to perform the abovementioned computer-implemented method, as well as to a non-invasive brain-machine interface system comprising this data processing device and biopotential electrode array, such as e.g. an EEG electrode array, an EMG electrode array, an ECG electrode array, and/or a SCR electrode array connected to the data processing device.

A third aspect of the present disclosure relates to a computer program and/or computer-readable medium comprising instructions which, when executed by a computer, cause the computer to perform the abovementioned computer-implemented method.

The above summary of some example embodiments is not intended to describe each disclosed embodiment or every implementation of the invention. In particular, selected features of any illustrative embodiment within this specification may be incorporated into an additional embodiment unless clearly stated to the contrary.

BRIEF DESCRIPTION OF THE DRAWINGS

The invention may be more completely understood in consideration of the following detailed description of various embodiments in connection with the accompanying drawings, in which:

- FIG. 1 shows a block diagram schematically illustrating a machine with a Brain-Machine Interface system incorporating a data processing device for obtaining continuous signals according to a first embodiment;
- FIG. 2 shows a schematic diagram of the data flow and data processing in the data processing device according to the first embodiment;
- FIG. 3 shows a block diagram schematically illustrating a machine with a Brain-Machine Interface system incorporating a data processing device for obtaining continuous signals according to a second embodiment;
- FIG. 4 shows a schematic diagram of the data flow and data processing in the data processing device according to the second embodiment; and
- FIG. 5 shows a block diagram schematically illustrating a machine with a Brain-Machine Interface system incorporating a data processing device for obtaining continuous signals according to a third embodiment.

While the invention is amenable to various modifications and alternative forms, specifics thereof have been shown by way of example in the drawings and will be described in detail. It should be understood, however, that the intention is not to limit aspects of the invention to the particular embodiments described. On the contrary, the intention is to cover all modifications, equivalents, and alternatives falling within the scope of the invention.

DETAILED DESCRIPTION

For the following defined terms, these definitions shall be applied, unless a different definition is given in the claims or elsewhere in this specification.

All numeric values are herein assumed to be preceded by the term "about", whether or not explicitly indicated. The term "about" generally refers to a range of numbers that one of skill in the art would consider equivalent to the recited value (i.e. having the same function or result). In many instances, the term "about" may be indicative as including numbers that are rounded to the nearest significant figure.

Any recitation of numerical ranges by endpoints includes all numbers within that range (e.g., 1 to 5 includes a.o. 1, 4/3, 1.5, 2, *e,* 2.75, 3, n, 3.80, 4, and 5).

Although some suitable dimension ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

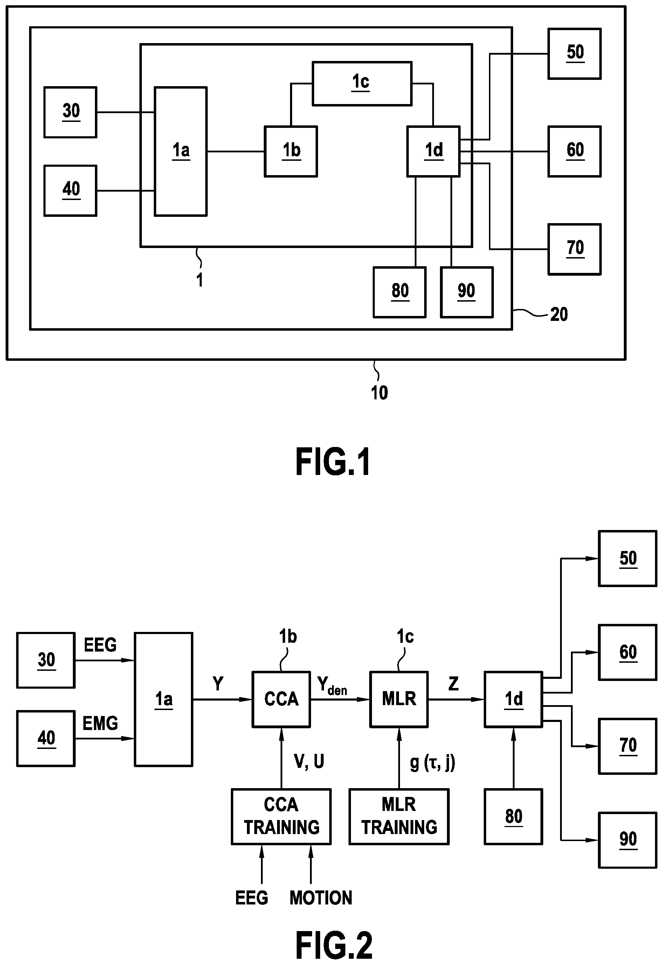

Fig. 1 shows a block diagram of a machine 10 with a non-invasive Brain-Machine Interface (BMI) system 20 comprising a data processing device 1 according to a first embodiment of the present disclosure. In this first embodiment, the machine 10 may be a vehicle comprising a steering system 50, a powertrain system 60 and/or a braking system 70. The powertrain system 60 may be, for instance, an internal combustion (IC) powertrain, an electric powertrain or a hybrid powertrain comprising both internal combustion and electric drive power sources.

The data processing device 1 may be connected to or comprise a data storage for storing a reference data set. The data processing device 1 may comprise an electronic circuit, a processor (shared, dedicated, or group), a combinational logic circuit, a memory that executes one or more software programs, and/or other suitable components that provide the described functionality. The data processing device 1 may additionally carry out further functions in the machine 10. For example, the control device may also act as the general purpose electronic control unit (ECU) of the machine 10.

In this first embodiment, the BMI system 20 may comprise, besides the data processing device 1, a non-invasive electroencephalography (EEG) electrode array 30 for collecting EEG signals from a human user. The EEG electrode array 30 may be connected to the data processing device 1 for transmitting those EEG signals to the data processing device 1. Alternatively or additionally to the EEG electrode array 30, the BMI system 20 may comprise a non-invasive electromyography (EMG) electrode array 40 for collecting EMG signals from the human user. The EMG electrode array 40 may be also connected to the data processing device 1 for transmitting those EMG signals to the data processing device 1.

The data processing device 1 may comprise a filter 1a, which may be for instance a $4^{th}$ order Butterworth filter, for filtering out EEG and/or EMG signal components above and/or below corresponding frequency thresholds, for instance a lower threshold of 0.1 Hz and an upper threshold of 8 Hz, and a denoising unit 1b, configured to perform a statistical correlation algorithm, such as a Canonical Correlation Analysis (CCA) algorithm, to identify and remove confounding non-brain components in the filtered signals received from the EEG and/or EMG electrode arrays 30, 40 through filter 1a so as to obtain denoised neural signals.

The data processing device 1 may further comprise a decoder unit 1c, configured to decode continuous signals from the denoised neural signals obtained by the denoising unit 1b. In particular, the decoder unit 1c may be configured to decode the continuous signals from the denoised neural signals using a Multiple Linear Regression (MLR) model. These continuous signals may in particular be continuous motor action command signals, that is, neural signals related to an intended motion of the user, for example an intended motion for actuating one or more control elements such as a wheel, pedal, handle, lever, joystick, paddle, etc. for operating the steering, braking and/or powertrain systems 50, 60, 70.

The data processing device 1 may also comprise a verification unit 1d, which may be configured to verify whether the continuous signals are acceptable, for example by comparing them to a permitted set of actions. The verification unit 1d may be connected to an advanced driver assistance system (ADAS) 80 which may be configured to update the permitted set of actions depending on the driving context. The verification unit 1d may also be connected to the steering, braking and/or powertrain systems 50, 60, 70, and may eventually be configured to prohibit their operation if a continuous signal received through the decoding unit 1c is determined to correspond to an inacceptable command. The verification unit 1d may additionally or alternatively be connected to a warning output unit 90, comprising for instance a visual display and/or a loudspeaker, for outputting a warning that the continuous signal corresponds to an inacceptable command. Alternatively or additionally to verifying whether the continuous signals are acceptable, the verification unit 1d may be configured to receive, e.g. from the ADAS 80, data concerning the response of the machine 10 to the user's inputs through the one or more control elements, and compare this response to the decoded continuous signals output by the decoder unit 1c to the verification unit 1d. This may be be used, for example, to evaluate the driveability of a vehicle during test drives.

Fig. 2 illustrates a method of operating the machine 10 using the non-invasive BMI system 20. This method may be stored in a computer-readable medium as a computer program comprising instructions that, when executed by a computer such as the data processing device 1, may cause the device to execute this method. In this method, the EEG and/or EMG electrode arrays 30, 40 may collect biopotential signals, such as EEG and/or EMG signals reflecting a user's neural activity, and transmit them to the data processing device 1, and more specifically to the denoising unit 1b, through the filter 1a. In the denoising unit 1b, confounding components may be identified in the EEG and/or EMG signals and removed from them, using a statistical correlation algorithm, such as a CCA algorithm.

A CCA algorithm can estimate a linear transformation of two multichannel datasets X and Y so as to minimise irrelevant variance. Given two datasets X and Y of size $T \times J_1$ and $T \times J_2$, the CCA algorithm can find linear transformations of both that make them maximally correlated. Specifically, the CCA algorithm can produce transformation matrices V and U, of respective sizes $J_1 \times J_0$ and $J_2 \times J_0$, where $J_0 < \min(J_1, J_2)$, whose product with, respectively, datasets X and Y results in transformed data matrices $X_V$ and $Y_U$. Each pair of a column of transformed data matrix $X_V$ and a corresponding column of transformed data matrix $Y_U$ forms a so-called canonical component (CC), and transformation matrices V and U are calculated so that the columns of the first CC are maximally correlated with each other, whereas those of each subsequent CC are also maximally correlated with each other but uncorrelated with the columns of the previous CCs. The first CC is thus the linear combination of X and Y with the highest possible correlation. The next pair of CCs are the most highly correlated combinations orthogonal to the first, and so on.

In a CCA algorithm as may be applied by the denoising unit 1b, the dataset Y may correspond to the filtered neural signal received by the denoising unit 1b and the dataset X may correspond to the confounding component to be removed from Y. This basic formulation of the CCA algorithm can capture an instantaneous interaction between stimulus representations and brain response. Applying the CCA algorithm may produce weighted sums $X_V(t) = \Sigma_i x_i(t) * v_i$ and $Y_U(t) = \Sigma_j y_j(t) * u_j$ that are maximally correlated, where each one of $x_i(t)$ and $y_j(t)$ represents one channel i, j of, respectively, datasets X and Y at each time point t, each one of $v_i$ and $u_j$ is a vector from, respectively, transformation matrices V and U, and each vector $v_i$ and $u_j$ representing the transformation weights for the corresponding channel i,j. After having estimated transformation matrices V and U and applied transformation matrix U to transform the dataset Y into transformed data matrix $Y_U$, the first component from $Y_U$, which corresponds to the signal component most correlated with the dataset X, may be removed from transformed data matrix $Y_U$ so as to obtain a denoised transformed data matrix $Y_{u,den}$ to which the inverse transformation matrix $U^{-1}$ may be applied to produce a multichannel dataset as a denoised neural signal $Y_{den}$.

When the continuous commands to be obtained are continuous motor action commands, this CCA algorithm may have been trained on an existing dataset of EEG and/or EMG signals and correlated motion data, so as to apply transformation matrices based on that dataset for performing the separation of the denoised brain signals from the confounding components in the EEG signals. More specifically, the existing dataset may include data from one or more users, possibly including the current user, and the motion data thereof may have been obtained using one or more EMG electrodes placed on relevant muscles, e.g. left and right deltoid muscles for steering control, one or more motion sensors such as accelerometers placed on positions of interest, e.g. neck and wrist for steering control, and/or one or more cameras performing visual motion capture, and be correlated to concurrent EEG and/or EMG signals from the same users. Once thus trained, the CCA algorithm may apply the resulting transformation matrices V and U for the separation of the denoised brain signals from the confounding components in the filtered EEG and/or EMG signals.

The denoised neural signals may then be transmitted from the denoising unit 1b to the decoder unit 1c, which may then proceed to decode, from these denoised neural signals, continuous signals which may be embedded therein. For this, the decoder unit 1c may use a Multiple Linear Regression (MLR) model in a backward-modelling system identification algorithm.

Specifically, starting from the denoised neural signal $Y_{den}$, the continuous signal Z can be represented in discrete time as:

$$Z(t) = \sum_{\tau,j} g(\tau, j) Y_{den}(t - \tau, j) + \varepsilon(t),$$

where Z(t) is a value of the continuous signal Z at time point t, $\tau$ represents a time lag of continuous signal Z, $g(\tau,j)$ are regression weights forming the MLR model and describing a linear transformation of the value $Y_{den}(t - \tau,j)$ of each channel j of the denoised neural signal $Y_{den}$ at earlier time points t - $\tau$, and $\varepsilon(t)$ represents the residual signal at time point t not explained by the model. The MLR model describes the linear transformation of the neural signal $Y_{den}$ for a specified range of values of time lag $\tau$ representing a forward-shifted time window. During training, the regression weights $g(\tau,j)$ can be estimated by minimising the mean-square error (MSE) between observed values Z(t) of continuous signal Z and the corresponding values $\hat{Z}(t)$ calculated by the linear transformation of the denoised neural signal $Y_{den}$:

$$min(\varepsilon(t)) = \sum_t [Z(t) - \hat{Z}(t)]^2$$

This minimisation problem can be solved by applying the Tikhonov regression closed formula:

$$g = (Z^T Z + \lambda I)^{-1} Z^T Y_{den}$$

where I is the identity matrix and $\lambda$ is a bias term or smoothing factor. Addition of this smoothing factor prevents overfitting to high-frequency noise along the low-variance dimensions. The forward-shifted time window, that is, the range of values for time lag $\tau$, and the bias term $\lambda$ may be selected to optimise the decoding metric of interest (e.g. MSE, decoding Pearson's correlation).

The MLR model may thus correlate continuous signals to denoised neural signals within an anticipating time window, shifted for instance 2-3 seconds in advance of the actual output of the continuous commands, for instance as motor actions. Consequently, the decoded continuous signals output by the decoder unit 1c to the verification unit 1d may anticipate an intended motion of the user, and in particular an intended motion for actuating one or more control elements such as a wheel, pedal, handle, lever, joystick, paddle, etc. for operating the steering, braking and/or powertrain systems 50, 60, 70, so that the verification unit 1d may verify whether they correspond to acceptable commands and prohibit them and/or output a warning before the user effectively outputs them to the steering, braking and/or powertrain systems 50, 60, 70 through his actuation of the one or more control elements, if they are not within the permitted set of actions, as received from instance from the ADAS 80, and/or compare those decoded continuous signals with the response of the machine 10 to the actuation of the control elements by the user.

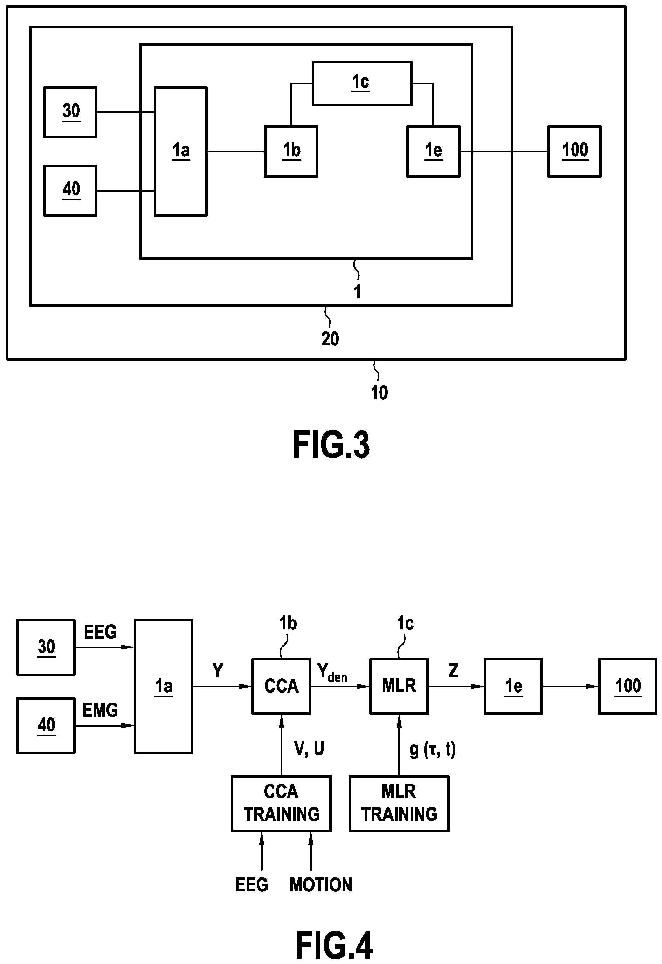

However, alternatively to operating the steering, braking and/or powertrain systems 50, 60, 70 with such control elements such as a wheel, pedal, handle, lever, joystick, paddle, the decoded continuous signals could be translated into machine operation commands. So for instance, in a second embodiment, illustrated in Figure 3, the machine 10 may be a prosthetic device, such as a partial exoskeleton, comprising a set of linear and/or angular actuators 100 and a non-invasive BMI system 20 comprising the data processing device 1 and EEG and/or EMG electrode arrays 30,40. All equivalent or analogous elements in this second embodiment will receive the same reference number as in the first embodiment.

As in the first embodiment, the data processing device 1 may additionally carry out further functions in the machine 10, it may also comprise an electronic circuit, a processor (shared, dedicated, or group), a combinational logic circuit, a memory that executes one or more software programs, and/or other suitable components that provide the described functionality and it may also be connected to or comprise a data storage for storing a reference data set. The data processing device 1 in this second embodiment may also comprise a filter 1a, denoising unit 1b and decoder unit 1c configured as in the first embodiment to obtain continuous signals, and in particular continuous motor action command signals, from EEG and/or EMG signals received from the EEG and/or EMG electrode arrays 30, 40. In this second embodiment, the transformation matrices of the CCA algorithm that may be applied by the denoising unit 1b and the MLR model used by the decoding unit 1c may be based on an existing dataset of EEG and/or EMG signals and correlated motion data from a set of multiple users, all representative of the current user (e.g. same age range, same right- or left-handedness), so as to reflect their common responses. Furthermore, in this second embodiment, alternatively to or in addition to the verification unit 1d of the first embodiment, the data processing device 1 may comprise an output unit 1e connected to the actuators 100 and configured to translate into machine operation commands the decoded continuous signals outputted by the decoder unit 1c and transmit them to the actuators 100 so as to operate the machine 10, possibly analogously to an impaired limb, as illustrated in Fig. 4. More advantageously than previous BMI systems that are based on motor imagery, the BMI system 20 according to this second embodiment would allow for the rehabilitation of the impaired neural circuitry and/or the operation of the prosthetic device in a natural way, i.e. without any additional cognitive effort required to the user.

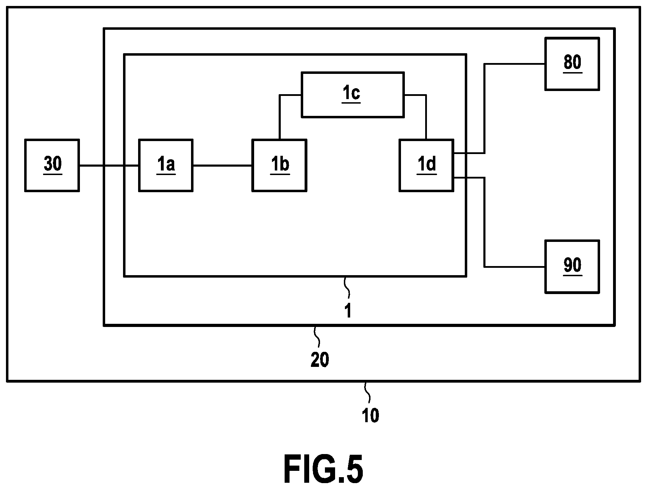

The continuous signals that can be decoded from denoised neural signals are not limited to motor action command signals. For example, a BMI system 20 according to a third embodiment of the present invention, illustrated in Fig. 5, may be configured to obtain a continuous attention signal from an EEG signal, and thus continuously monitor the user's attention, and eventually issue a warning and/or switch to a higher user assistance level and/or an automated mode if the user's attention drops beneath a threshold. As in the first embodiment, the machine 10 may in particular be a vehicle, and the user the vehicle's driver. All equivalent or analogous elements in this second embodiment will receive the same reference number as in the first and second embodiments.

As in the first and second embodiments, the data processing device 1 may additionally carry out further functions in the machine 10, it may also comprise an electronic circuit, a processor (shared, dedicated, or group), a combinational logic circuit, a memory that executes one or more software programs, and/or other suitable components that provide the described functionality and it may also be connected to or comprise a data storage for storing a reference data set. The data processing device 1 in this third embodiment may also comprise a filter 1a, denoising unit 1b and decoder unit 1c configured as in the first and second embodiments to obtain continuous signals, and in particular continuous attention signals, from EEG signals received from the EEG electrode array 30. In this third embodiment the denoising unit 1b may apply a CCA algorithm with transformation matrices based on an existing dataset of EEG signals and correlated motion, acoustic, vision, and/or other biopotential data from one or more users, possibly including the current user. The correlated motion data may have been obtained using one or more EMG electrodes, one or more motion sensors such as accelerometers placed on positions of interest, and/or one or more cameras performing visual motion capture, the correlated acoustic data may have been obtained using one or more microphones, and the correlated vision data may have been obtained using one or more cameras covering the corresponding user's field of vision. The denoising unit 1b may thus use the resulting transformation matrices to remove not only confounding motor but also aural and/or vision components from the filtered EEG signals to obtain a residual neural signal corresponding to a continuous attention signal. Other correlated data which may be used in the determination of the transformation matrices of the CCA algorithm include other biopotential data such as e.g. ECG data and/or SCR data. Furthermore, in this third embodiment, the regression weights of the MLR model applied by the decoder unit 1c may be based on a correlation between these residual neural signals obtained by the denoising unit 1b and a context, and in particular a driving context, as perceived by an ADAS 80 which may be connected to the decoder unit 1c. Specifically, the decoder unit 1c may thus apply the MLR model to determine the coupling between the residual neural signals obtained by the denoising unit 1b and an expectation vector consisting of the priors of statistical algorithms applied by the ADAS 80 for the prediction of upcoming events during driving. The decoder unit 1c may be further connected to a verification unit 1d, within the data processing device 1, configured to determine whether the driver's attention, as measured by the decoder unit 1c through the correlation between the residual neural signals and the upcoming driving events, is below an acceptable threshold. The BMI system 20 may further comprise a warning output unit 90, comprising for instance a visual display and/or a loudspeaker, connected to the verification unit 1d and configured to output a warning if the verification unit 1d determines that the driver's attention, as measured by the correlation between the residual neural signals and the upcoming driving events, is below an acceptable threshold. Alternatively or additionally to this connection, the verification unit 1d may be connected to an ADAS 80, within the BMI system 20, and configured to switch this ADAS 80 to a higher driver assistance level and/or an automated driving mode if it is determined that the driver's attention is below said acceptable threshold.

In a first variant of this third embodiment, the EEG electrode array 30 may be replaced with another type of biopotential electrode array, such as an EMG electrode array or even an SCR and/or ECG electrode array. In this case, the denoising unit 1b may apply a CCA algorithm with transformation matrices based on an existing dataset of EMG, SCR and/or ECG signals and correlated motion, acoustic, vision, and/or other biopotential data from one or more users, possibly including the current user, to remove the confounding components and obtain a residual neural signal corresponding to the continuous attention signal.

In a second variant of this third embodiment, the machine 10 may be a vehicle capable of highly automated driving, with a highly automated driving system instead of an ADAS. This highly automated driving system may be connected to the steering, braking and/or powertrain systems 50, 60, 70 to command their operation and may configured to perform highly automated driving in various different modes. In this case, rather than to continuous attention signals, the residual neural signals may be correlated for instance to continuous comfort signals and a vehicle context, the decoder unit 1c may be configured to decode these continuous comfort signals through this correlation between the residual neural signals and continuous comfort signals, taking into account contextual information transmitted to the decoder unit 1c by the highly automated driving system, and the verification unit 1e may be replaced by a driving mode switching unit configured to switch the highly automated driving system between the different modes, depending on a value of the continuous signal outputted by the decoding unit 1c and the contextual information.

Those skilled in the art will recognize that the present invention may be manifested in a variety of forms other than the specific embodiments described and contemplated herein. Accordingly, departure in form and detail may be made without departing from the scope of the present invention as described in the appended claims.

## Claims

1. A computer-implemented method for obtaining continuous signals from biopotential signals, comprising the steps of:

    separating confounding components from the biopotential signals by using a statistical correlation analysis algorithm to obtain denoised neural signals; and
    decoding the continuous signals from the denoised neural signals.

2. The computer-implemented method according to claim 1, wherein the continuous signals are continuous motor action commands.

3. The computer-implemented method according to any one of claims 1 or 2, wherein the statistical correlation analysis algorithm is a Canonical Correlation Analysis algorithm.

4. The computer-implemented method according to any one of claims 1 to 3, wherein the statistical correlation analysis algorithm is performed using a model based on an existing dataset of biopotential signals and correlated motion, vision, acoustic and/or other biopotential data.

5. The computer-implemented method according to claim 4, wherein the correlated motion data include EMG data, motion sensor data, visual motion capture data.

6. The computer-implemented method according to any one of the previous claims, wherein the step of decoding continuous signals from the denoised neural signals is performed using a Multiple Linear Regression model.

7. The computer-implemented method according to claim 6, wherein the Multiple Linear Regression model correlates continuous signals to denoised neural signals within an anticipating time window.

8. The computer-implemented method according to any one of the previous claims, further comprising a step of filtering the biopotential signals before the step of separating the confounding components thereof.

9. The computer-implemented method according to any one of claims 1 to 8, further comprising a step of operating a machine (10) using the continuous signals as command signals.

10. The computer-implemented method according to claim 9, wherein the machine (10) is a vehicle.

**11.** The computer-implemented method according to claim 9, wherein the machine (10) is a robotic manipulator.

**12.** The computer-implemented method according to claim 9, wherein the machine (10) is a prosthetic device.

**13.** The computer-implemented method according to any one of claims 1 to 12, further comprising a step of verifying whether the continuous signals correspond to continuous commands within a set of acceptable continuous commands.

**14.** The computer-implemented method according to any one of claims 1 to 13, further comprising a step of comparing the continuous signals to a response of a machine (10) operated by a human user generating the biopotential signals.

**15.** A data processing device (1) comprising a processor configured to perform the computer-implemented method according to any one of claims 1 to 14.

**16.** A non-invasive brain-machine interface system (20) comprising a data processing device (1) according to claim 15 and an EEG electrode array (30) and/or an EMG electrode array (40) connected to the data processing device (1).

**17.** A computer program comprising instructions which, when executed by a computer, cause the computer to perform the computer-implemented method according to any one of claims 1 to 14.

**18.** A computer-readable medium comprising instructions which, when executed by a computer, cause the computer to carry out the computer-implemented method according to any one of claims 1 to 14.

FIG.1

FIG.2

**FIG.3**

**FIG.4**

FIG.5

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/330404 A1 (ABDELGHANI MOHAMED [US] ET AL) 6 November 2014 (2014-11-06) <br> * figures 7A,7B,8,9,14,15 * <br> * paragraph [0059] * <br> * paragraph [0068] - paragraph [0073] * <br> ----- | 1,2,4-18 | INV. <br> A61B5/00 <br> A61B5/04 <br> A61B5/0476 <br> G06F3/01 <br> A61F2/72 |
| X | KARTSCH VICTOR ET AL: "BioWolf: A Sub-10-mW 8-Channel Advanced Brain-Computer Interface Platform With a Nine-Core Processor and BLE Connectivity", IEEE TRANSACTIONS ON BIOMEDICAL CIRCUITS AND SYSTEMS, IEEE, US, vol. 13, no. 5, 1 October 2019 (2019-10-01), pages 893-906, XP011754441, ISSN: 1932-4545, DOI: 10.1109/TBCAS.2019.2927551 [retrieved on 2019-11-05] <br> * figures 1-3,8 * <br> * page 895, column 2, line 29 - page 896, column 1, line 11 * <br> * page 899, column 2, line 51 - page 900, column 2, line 12 * <br> ----- | 1,3,4,8, 15-18 | A61B5/0488 |
| A | DOMEN NOVAK ET AL: "A survey of sensor fusion methods in wearable robotics", ROBOTICS AND AUTONOMOUS SYSTEMS, vol. 73, 1 November 2015 (2015-11-01), pages 155-170, XP055709883, AMSTERDAM, NL ISSN: 0921-8890, DOI: 10.1016/j.robot.2014.08.012 <br> * figure 1 * <br> * page 156, column 1, line 32 - column 2, line 55 * <br> * page 159, column 1, line 24 - line 42 * <br> ----- <br> -/-- | 6,7 | TECHNICAL FIELDS SEARCHED (IPC) <br> A61B <br> G06F <br> A61F |

The present search report has been drawn up for all claims

| Place of search | Date of completion of the search | Examiner |
|---|---|---|
| The Hague | 5 November 2020 | Oancea, A |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

**EUROPEAN SEARCH REPORT**

Application Number

EP 20 17 5553

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 3 063 601 A1 (COMMISSARIAT À L EN ATOMIQUE ET AUX EN ALTERNATIVES [FR]) 7 September 2016 (2016-09-07) * figure 1 * * paragraph [0015] - paragraph [0018] * * paragraph [0035] - paragraph [0043] * ----- | 13 | |

TECHNICAL FIELDS
SEARCHED      (IPC)

The present search report has been drawn up for all claims

| Place of search | Date of completion of the search | Examiner |
|---|---|---|
| The Hague | 5 November 2020 | Oancea, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 17 5553

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2020

| Patent document cited in search report | | | Publication date | Patent family member(s) | | | Publication date |
|---|---|---|---|---|---|---|---|
| US 2014330404 | A1 | | 06-11-2014 | NONE | | | |
| EP 3063601 | A1 | | 07-09-2016 | EP | 3063601 | A1 | 07-09-2016 |
| | | | | US | 2016282941 | A1 | 29-09-2016 |
| | | | | WO | 2015063535 | A1 | 07-05-2015 |

EPO FORM P0459

For more details about this annex : see Official Journal of the European Patent Office, No. 12/82

**EP 3 912 547 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3556429 A1
- US 9824607 B1
- WO 2015003118 A1
- US 20140058528 A1